# EUROPEAN PATENT APPLICATION

(11) **EP 1 990 333 A1**
(43) Date of publication of application: **12.11.2008**
(21) Application number: 07108042.8
(22) Date of filing: 11.05.2007
(51) Int. Cl.: C07C 209/50, C07C 211/30

(54) **Process for the preparation of cinacalcet hydrochloride**

(71) Applicant: SANDOZ AG, 4056 Basel (CH)
(72) Inventor: The designation of the inventor has not yet been filed
(74) Representative: Fauss-Berghus, Waltraud

(57) **Abstract**

The present invention relates to a process for the preparation of cinacalcet hydrochloride which is industrially feasible and commercially viable. The synthesis of cinacalcet hydrochloride is carried out by the condensation of 3-trifluromethylphenyl propionic acid with R-(+)-1-(lnaphthyl)ethylamine.

## Description

### FIELD OF THE INVENTION

The present invention relates to a process for the preparation of cinacalcet, (R)-α-methyl-N-[3-[3-(trifluoromethyl) phenyl] propyl]-1-naphthalenemethane amine which is industrially feasible and commercially viable.

### BACKGROUND OF THE INVENTION

Cinacalcet hydrochloride is a calcimimetic agent which increases the sensitivity of the calcium sensing receptor to activation by extracellular calcium. It is indicated for the treatment of secondary hyperthyroidism in patients with chronic renal disease on dialysis and also to treat hypercalcemia in patients with parathyroid carcinoma.

Calcimimetics are a class of orally active, small molecules that decrease the secretion of parathyroid hormone [PTH] by activating calcium receptors. The secretion of PTH is normally regulated by the calcium-sensing receptor. Calcimimetic agents increase the sensitivity of this receptor to calcium, which inhibits the release of parathyroid hormone, and lowers parathyroid hormone levels within a few hours. Calcimimetics are used to treat hyperparathyroidism, a condition characterized by the over-secretion of PTH that results when calcium receptors on parathyroid glands fail to respond properly to calcium in the bloodstream. Elevated levels of PTH, an indicator of secondary hyperparathyroidism, are associated with altered metabolism of calcium and phosphorus, bone pain, fractures, and an increased risk for cardiovascular death.

U.S. Patent No.6,313,146 (the '146 patent) describes the molecules which can modulate one or more inorganic ion receptor activities in the body, preferably a calcium receptor. The '146 patent claims cinacalcet generically. The method of preparation of the claimed compounds involves synthesis using sodium cyanoborohydride as the catalyst.

U.S. Patent No. 6,211,244 (the '244 patent) discloses calcium receptor-active compounds related to cinacalcet and methods of making such compounds. In accordance with the'244 patent, cinacalcet may be produced by reacting 1-acetyl naphthalene with 3-[3-(trifluoromethyl)phenyl] propylamine in the presence of titanium isopropoxide to produce an imine corresponding to cinacalcet, followed by treatment with methanolic sodium cyanoborohydride and resolution of the racemic cinacalcet base by chiral liquid chromatography, according to Scheme I.

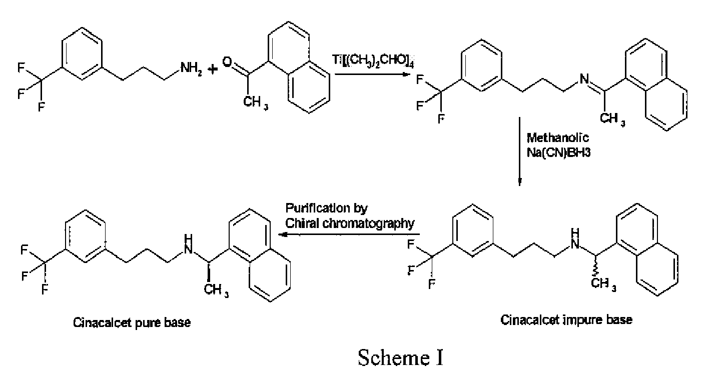

Similarly, using the process disclosed in the '244 patent , the desired cinacalcet enantiomer may be produced by reacting (R)-1-(1-naphthyl)ethylamine with 3-[3-(trifluoromethyl)phenyl] propionaldehyde in the presence of titanium isopropoxide, to produce the imine that corresponds to cinacalcet, followed by treatment with ethanolic sodium cyanoborohydride, according to Scheme II below.

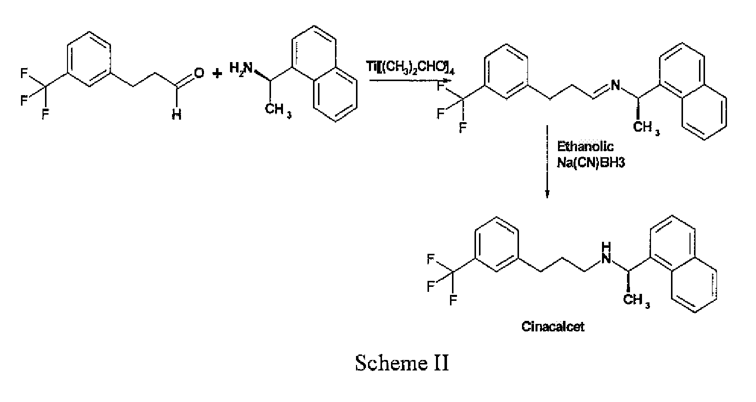

The '244 patent discloses an additional process for the synthesis of cinacalcet. This process involves treating 3-trifluoromethylcinnamonitrile, which can be prepared as disclosed in U.S. Patent 4,966,988, with diisobutyl aluminum hydride, followed by treating the intermediate aluminum-imine complex with (R)-1-(1 -naphthyl)ethylamine, and reducing the intermediate imine with ethanolic sodium cyanoborohydride, according to Scheme III below.

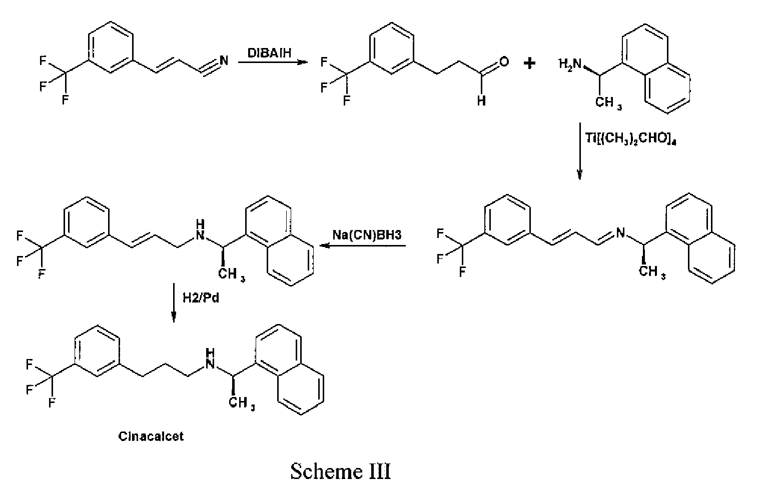

U. S. Patent No. 6,011,068 (the patent '068 patent) is related to compounds which modulate one or more activities of an inorganic ion receptor and methods for treating diseases or disorders by modulating inorganic ion receptor activity. The '068 patent claims cinacalcet generically. The claimed compounds are synthesized using sodium cyanoborohydride and titanium isopropoxide as the catalyst.

European patent no. 1203761 claims cinacalcet and its salt specifically. Although this patent does not mention a process for the synthesis of cinacalcet, various uses of cinacalcet are claimed.

European Patent no.1281702 claims cinacalcet generically. The method for the synthesis of the claimed compounds uses titanium isopropoxide as the catalyst.

PCT patent application WO2006/125026 describes a method to synthesize cinacalcet hydrochloride. The application describes a process for the synthesis of cinacalcet by converting 3-[3-(trifluromethyl) phenyl] propan-1-ol to a compound with a good leaving group and further reacting it with R- 1-Naphthylethylamine to from cinacalcet base. The reagents described in the patent application which have good leaving groups are thionyl halide, aliphatic sulfonyl halide and aromatic sulfonyl halide.

An alternative process for the preparation of cinacalcet base and cinacalcet salt, which is more direct, gives quantitative yield, is environmental friendly and applicable to industrial scale production, is desirable. The present invention provides such an alternative.

### SUMMARY OF THE INVENTION

The present invention relates to a process for the preparation of cinacalcet hydrochloride which is industrially feasible and commercially viable. The synthesis of cinacalcet hydrochloride is carried out by the condensation of 3-trifluromethylphenyl propionic acid with R-(+)-1-(1-naphthyl)ethylamine to obtain the corresponding amide, and further reduction of the amide to the corresponding amine using BF₃Et₂O/NaBH₄.

### DETAILED DESCRIPTION OF THE INVENTION

The present invention relates to the synthesis of cinacalcet hydrochloride, which is carried out by the condensation of 3-(trifluromethylphenyl) propionic acid with R-(+)-1-(1-naphthyl)ethylamine to obtain the corresponding amide and further reduction of the amide to the corresponding amine.

The process of the present invention could be used for the preparation of various salts, forms and solvates of cinacalcet.

The present invention discloses a process for the synthesis of cinacalcet hydrochloride comprising the steps of:
(a)hydrogenating 3- (trifluoromethyl) cinnamic acid in the presence of Pd/C, hydrogen and solvent to form 3-(trifluoromethylphenyl) propionic acid;
(b) reacting 3-(trifluoromethylphenyl) propionic acid with ethyl chloroformate to give a reactive *in situ* intermediate;
(c) condensing the reactive intermediate *in situ* with R-(+)-1-(1-naphthyl)ethylamine in a solvent to obtain the corresponding amide;
(d) reducing the amide of step (c) using a reducing agent in a solvent to form the corresponding amine; and
(e) treating the amine of step (d) with hydrochloric acid in a solvent to form cinacalcet hydrochloride.

In the present invention, step (a) is directed towards the hydrogenation of 3-(trifluoro methyl) cinnamic acid using Pd/C as the catalyst. The solvent of step (a) includes, but are not limited to, methanol ,ethanol and tetrahydrofuran.

In the present invention, step (b) involves reacting 3-(trifluoromethylphenyl) propionic acid with ethyl chloroformate to give a mixed anhydride in solution

The solvent used in step (b) is ethyl acetate, tetrahydrofuran or toluene. In one embodiment, of the present invention the solvent of step (b) is ethyl acetate.

The reaction temperature of step (b) ranges from 0-50°C. In one embodiment, the reaction temperature ranges from 0-5°C.

The bases used in step (b)include but are not limited to, triethyl amine, isopropyl ethyl amine , 4-(dimethyl amino) pyridine, potassium carbonate.

In the present invention, step (c) involves condensing the in-situ intermediate with R-(+)-1-(1-naphthyl)ethylamine in a solvent to obtain the corresponding amide

The solvent used in step (c) is ethyl acetate or tetrahydrofuran. In one embodiment, of the present invention the solvent of step (c) is ethyl acetate.

The reaction temperature of step (c) ranges from 0-70°C. In one embodiment, the reaction temperature ranges from 0-5°C.

In the present invention, step (d) involves reducing the amide of step (c) using a reducing agent in a solvent to form the corresponding amine which is cinacalcet base;

The reducing agent of step (d) includes, but is not limited to, sodium borohydride and boron trifluoride-etherate, sodium borohydride -iodine, lithium aluminum hydride-tetrahydrofuran, sodium borohydride-phosphorous trichloride or sodium borohydride - acetic acid. In one embodiment of the present invention the reducing agent of step (c) is sodium borohydride and boron trifluoride-etherate.

The solvent used in step (d) is tetrahydrofuran.

The reaction temperature of step (d) ranges from 0-100°C. In another embodiment, the reaction temperature ranges from 20-80°C.

The reaction time for the step (d) ranges from 2 to 30 hours. In further embodiment the reaction time ranges from 2- 8 hours.

In the present invention, step (e) involves the conversion of cinacalcet free base to cincacalcet hydrochloride.

The solvent of step (e) includes, but is not limited to, diisopropyl ether and diethyl ether

The reaction temperature of step (e) ranges from 0-40°C. In another embodiment, the reaction time ranges from 0-30°C.

In the present invention, the method for the preparation of N-(1-Naphthalen-1-yl-ethyl)-3-(3-teifluoromcthyl-phenyl)-propionamide by condensation of 3-(trifluoromethylphenyl) propionic acid with R-(+)-1-(1-Naphthyl)ethylamine using ethyl chloroformate is facile and unique.

The process for the present invention results in the formation of crystalline form of cinacalcet hydrochloride.

The cinacalcet base may contain a small amount of the intermediate of the preceding steps.

The present invention provides a process, which is direct, gives quantitative yield, is environment friendly, and can be applied to industrial scale production.

### Examples 1 to 4 [Scheme IV]

Scheme IV depicts the general process for the synthesis of cinacalcet. The various parameters in each steps is described in the foregoing examples 1 to 4.

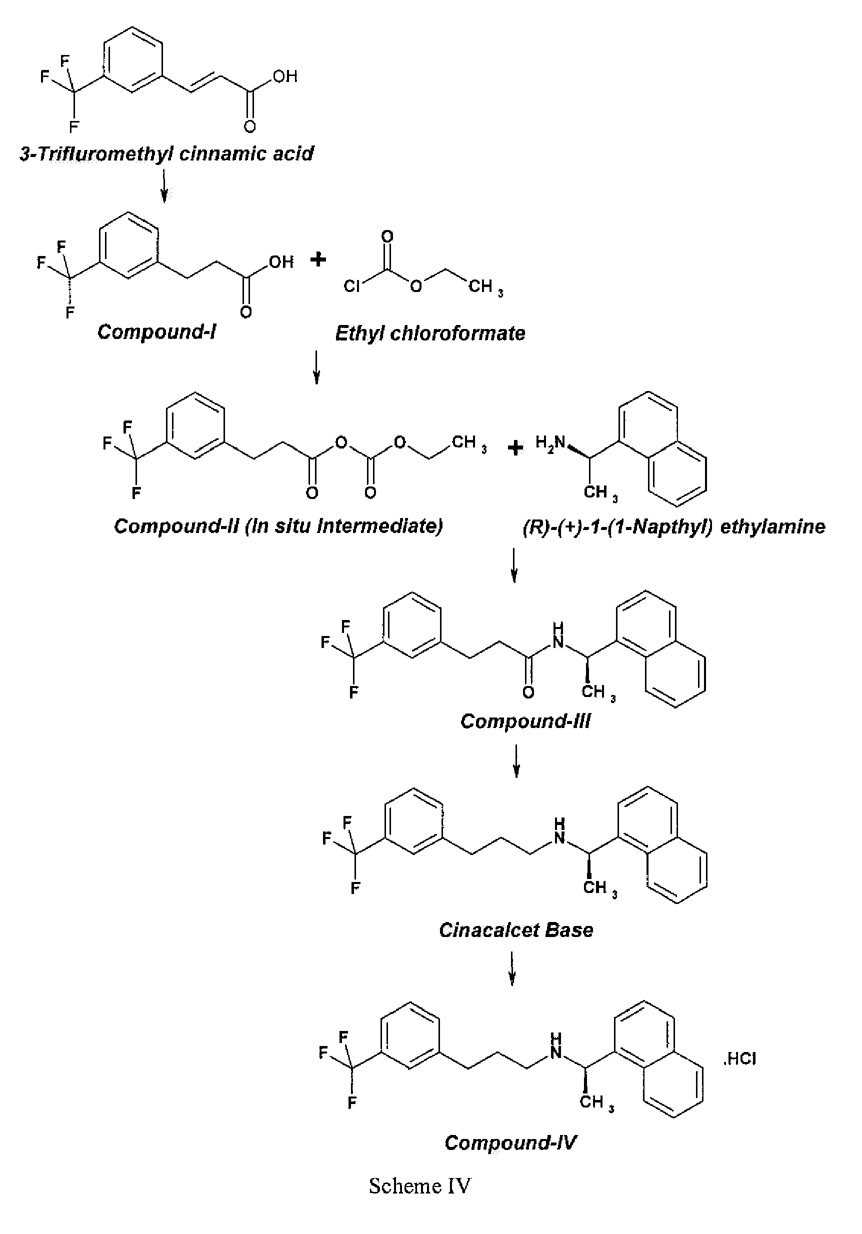

### Example 1

### Step (i) Preparation of 3-(3-Trifluoromethyl-phenyl)-propionic acid [Compound -I]:

Methanolic solution of 3- (Trifluoromethyl) cinnamic acid (30.0g in 270.0 mL of methanol) was hydrogenated (5.0kg/cm² of H₂) in the presence of 3.0gm palladium on carbon (10% w/w of 3- (trifluoromethyl)cinnamic acid) for 3 hours at room temperature. Then the catalyst was filtered out and the solvent was evaporated to dryness to give 28.4 g of titled compound-I.

### Analytical Data

¹H NMR (DMSO-d₆, 400 MHz) of 3- (Trifluoromethyl) cinnamic acid: δ 6.714-6.754 (doublet, 2H); δ 7.638-7.721 (broad multiplet, 2H); δ 7.758-7.777 (doublet, 1H); δ 8.030-8.050 (doublet, 1H); δ 8.095 (singlet, 1H); δ 12.607 (singlet, 1H).

¹H NMR (CDCl₃, 400 MHz) of compound-I: δ 2.696-2.735 (triplet, 2H); δ 3.003-3.041 (triplet, 2H); δ 7.405-7.422 (broad multiplet, 3H); δ 7.475-7.492 (singlet,2H).

### Step (ii) Preparation of N-(1-Naphthalen-1-yl-ethyl)-3-(3-tritluoromethyl-phenyl)-propionamide [Compound-III]:

20.0gm of compound-I was dissolved in ethyl acetate and 10.20gm of triethylamine was added and stirred for 15.0 min. A solution of 9.95 gm of ethyl chloroformate in ethyl acetate was prepared and was added slowly in the reaction mass at 0-5°C. The reaction mass was stirred at 0-5°C for 2 hours to form mixed anhydride[ compound-II] . A solution of 17.27gm of (R)-(+)-1-(1-Napthyl) ethyl amine in ethyl acetate was prepared and added to the reaction mass at 0-5°C. The reaction mixture was stirred at 0-5°C for 1 hour .Water was added to this reaction mixture and stirred and the organic phase was separated. The organic phase was washed with 10% hydrochloric acid, water, 10% sodium bicarbonate solution and water successively. The organic phase was dried over sodium sulphate and the solvent was evaporated under vacuum to give crude titled compound-III. To this residue, n-hexane was added and heated the reaction mass to reflux for 30.0 min, cooled gradually to room temperature and stirred for 30.0 min. It was filtered and washed with chilled n-hexane. Wet compound on drying yields 26.0 gm of titled compound-III. Mass spectra is a depicted in figure I

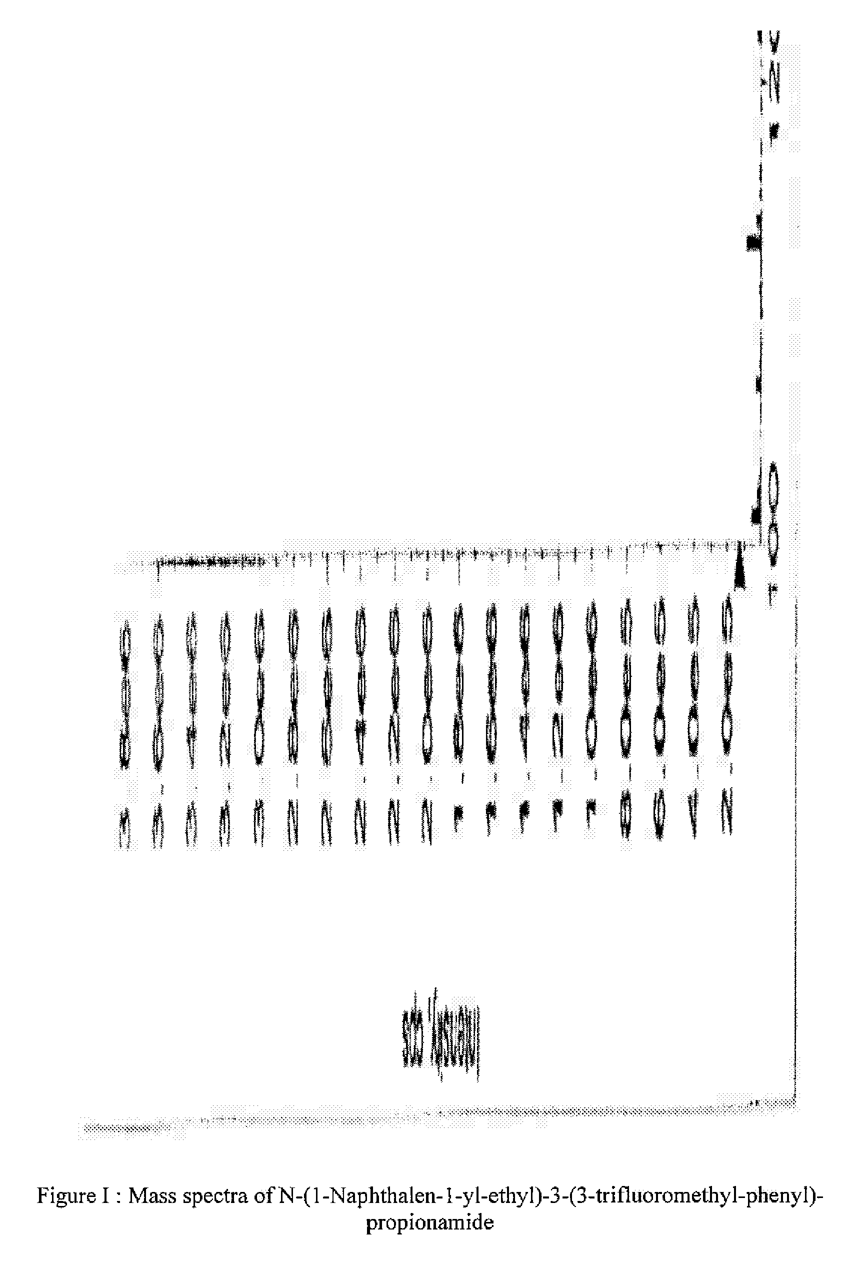

### Analytical Data-

¹HNMR (CDCl₃, 400 MHz) of compound-II: δ 1.213-1.248 (triplet, 3H); δ 2.627-2.665 (triplet, 2H); δ 2.994-3.032 (triplet, 2H); δ 4.102-4.156 (quartet, 2H); δ 7.395-7.472 (broad multiplet, 4H).

¹HNMR (CDCl₃, 400 MHz) of compound-III: δ 1.727-1.837 (doublet, 3H); δ 1.754-2.083 (broad multiplet, 2H); δ 2.653-2.713 (broad multiplet, 1H); δ 2.873-2.994 (broad multiplet, 1H); δ 5.183-5.243 (quartet, 1H); δ 7.146-7.321 (broad multiplet, 4H); δ 7.554-7.663 (broad multiplet, 3H); δ 7.809-8.007 (broad multiplet, 4H).

### Step (iii) Preparation of (R)-α-methyl-N-[3-[3-(trifluoromethyl)phenyl]propyl]-1-naphthalene methanamine [Cinacalcet base]:

Compound-III (15.0g) was dissolved in tetrahydrofuran and 7.5g of sodium borohydride was added to the solution. The reaction mass was cooled to 5°C and 30.0mL of boron trifluoride-etherate was added drop wise. The reaction mass was then heated to 30°C and stirred for 20 h. The reaction mass was quenched in 10% aqueous hydrochloric acid solution. The reaction mass was then refluxed for 2 h and cooled to 30°C. To this reaction mixture was added ethyl acetate. Phase separation was done. The organic phase was washed with water, 10% sodium bicarbonate solution and water successively. The organic phase was dried over sodium sulphate and the solvent was evaporated to dryness to obtain cinacalcet free base.

### Step (iv) Conversion of cinacalcet base into its hydrochloride salt.[Compound IV]

The cinacalcet free base of step (iii) was dissolved in diisopropyl ether and treated with 1M hydrochloric acid in diethyl ether. The resulting precipitate was collected, washed with diisopropyl ether and air dried to give 8.8 g of titled compound-IV (cinacalcet hydrochloride).

### Analytical Data-

¹H NMR (CDCl₃, 400 MHz) of compound-IV: δ 1.828-1.845 (doublet, 3H); δ 1.994-2.112 (broad multiplet, 2H); δ 2.471-2.598 (multiplet, 2H); δ 2.663-2.733 (broad multiplet, 1H); δ 2.873-2.994 (broad multiplet, 1H); δ 5.243-5.293 (quartet, 1H); δ 7.157-7.331 (broad multiplet, 4H); δ 7.564-7.664 (broad multiplet, 3H); δ 7.809-8.007 (broad multiplet, 4H).

### Example 2

### Step (i) Preparation of 3-(3-Trifluoromethyl-phenyl)-propionic acid [Compound -I]:

10.0gm of 3- (Trifluoromethyl) cinnamic acid and ethanol is charged. To this reaction mixture a slurry of 1.0gm palladium on carbon in 5.0 ml of ethanol is added. The reaction mixture is stirred at room temperature for 3 hours in a hydrogenator under hydrogen at a pressure of 5.0kg/cm². The catalyst is filtered and the solvent is evaporated to dryness to get title compound-I.

### Step (ii) Preparation of N-(1-Naphthalen-1-yl-ethyl)-3-(3-trifluoromethyl-phenyl)-propionamide [Compound-III]:

Dissolve 14.0gm of compound-I in tetrahydrofuran and cool to 10°C.Add 18.2gm of triethylamine and then 9.8gm of ethyl chloroformate slowly. Stir the reaction mass at 30°C for 2 hours. To this reaction mixture, add 12.0gm of (R)-(+)-1-(1-Napthyl) ethyl amine at 30°C and stir for 1 hour. Heat the reaction mass to reflux and stir at reflux for 2 hours. Cool the reaction mass to 30°C and distill out tetrahydrofuran below 40°C. To this reaction mixture add water and ethyl acetate. Separate the organic phase. Wash the organic phase with 10% hydrochloric acid, water, 10% sodium bicarbonate solution and water successively. Dry the organic phase over sodium sulphate and then evaporate the solvent under reduced pressure to get title compound III.

The bases which could be used in this step are included in the table

| Bases |
|---|
| Isopropyl ethyl amine |
| 4-(dimethyl amino) pyridine |
| Potassium carbonate |

### Step (iii) Preparation of (R)-α-methyl-N-[3-[3-(trifluoromethyl)phenyl]propyl]-1-naphthalene methanamine [Cinacalcet base]:

Compound-III (15.0g) is dissolved in tetrahydrofuran and 7.5g of sodium borohydride is added to the solution. The reaction mass is cooled to 5°C and 30.0mL of boron trifluoride-etherate is added drop wise. The reaction mass is then heated to 30°C and stirred for 5 h. The reaction mass is quenched in 10% aqueous hydrochloric acid solution. The reaction mass is then refluxed for 2 h and cooled to 30°C. Ethyl acetate is added to this reaction mixture. Phase separation is carried out. The organic phase is washed with water, 10% sodium bicarbonate solution and water successively. The organic phase is dried over sodium sulphate and the solvent is evaporated to dryness to obtain cinacalcet free base.

### Step (iv) Conversion of cinacalcet base into its hydrochloride salt.[Compound IV]

The cinacalcet free base of step (iii) is dissolved in diisopropyl ether and treated with 1M hydrochloric acid in diethyl ether. The resulting precipitate is collected, washed with diisopropyl ether and air dried to give 8.8 g of titled compound-IV (cinacalcet hydrochloride).

### Example 3

### Step (i) Preparation of 3-(3-Trifluoromethyl-phenyl)-propionic acid [Compound -I]:

10.0gm of 3- (Trifluoromethyl) cinnamic acid and ethanol is charged. To this reaction mixture a slurry of 1.0gm palladium on carbon in 5.0 ml of ethanol is added. The reaction mixture is stirred at room temperature for 3 hours in a hydrogenator under hydrogen at a pressure of 5.0kg/cm². The catalyst is filtered and the solvent is evaporated to dryness to get title compound-I.

### Step (ii) Preparation of N-(1-Naphthalen-1-yl-ethyl)-3-(3-trifluoromethyl-phenyl)-propionamide [Compound-III]:

Dissolve 14.0gm of compound-I in tetrahydrofuran and cool to 10°C.Add 18.2gm of triethylamine and then 9.8gm of ethyl chloroformate slowly. Stir the reaction mass at 30°C for 2 hours. To this reaction mixture, add 12.0gm of (R)-(+)-1-(1-Napthyl) ethyl amine at 30°C and stir for 1 hour. Heat the reaction mass to reflux and stir at reflux for 2 hours. Cool the reaction mass to 30°C and distill out tetrahydrofuran below 40°C. To this reaction mixture add water and ethyl acetate. Separate the organic phase. Wash the organic phase with 10% hydrochloric acid, water, 10% sodium bicarbonate solution and water successively. Dry the organic phase over sodium sulphate and then evaporate the solvent under reduced pressure to get title compound III.

### Step (iii) Preparation of (R)-α-methyl-N-[3-[3-(trifluoromethyl)phenyl]propyl]-1-naphthalene methane amine [Cinacalcet base]:

Dissolve 5.0gm of compound-III in tetrahydrofuran and add 17.0gm Lithium aluminium hydride to the solution under nitrogen. Cool the reaction mass to 10°C ,stir at 10°C for 2 hours and reflux for 2 hrs. Cool the reaction mass to 10°C and slowly add water at 10°C. Stir for 30.0 min. Distill out tetrahydrofuran below 40°C. To this reaction mixture add ethyl acetate. Separate the organic phase. Wash the organic phase with 10% sodium bicarbonate solution and water successively. Dry the organic phase over sodium sulphate and then evaporate the solvent under vacuum to get title compound IV.

The reducing agent and the reaction time which could be used in this step are included in the following table

| Reducing agent | Reflux time |
|---|---|
| NaBH4- acetic acid | 5 hours |
| NaBH4-iodide | 18 hours |

### Step (iv) Conversion of cinacalcet base into its hydrochloride salt.[ compound IV]

The cinacalcet free base of step (c) was dissolved in diisopropyl ether and treated with 1M hydrochloric acid in diethyl ether. The resulting precipitate was collected, washed with diisopropyl ether and air dried to afford 8.8 g of titled compound-IV (cinacalcet hydrochloride).

### Example 4

### Step (i) Preparation of 3-(3-Trifluoromethyl-phenyl)-propionic acid [Compound -I]:

10.0gm of 3- (Trifluoromethyl) cinnamic acid and ethanol is charged. To this reaction mixture a slurry of 1.0gm palladium on carbon in 5.0 ml of ethanol is added. The reaction mixture is stirred at room temperature for 3 hours in a hydrogenator under hydrogen at a pressure of 5.0kg/cm². The catalyst is filtered and the solvent is evaporated to dryness to get title compound-I.

### Step (ii) Preparation of N-(1-Naphthalen-1-yl-ethyl)-3-(3-trifluoromethyl-phenylpropionamide [Compound-III]:

Dissolve 14.0gm of compound-I in tetrahydrofuran and cool to 10°C.Add 18.2gm of triethylamine and then 9.8gm of ethyl chloroformate slowly. Stir the reaction mass at 30°C for 2 hours. To this reaction mixture, add 12.0gm of (R)-(+)-1-(1-Napthyl) ethyl amine at 30°C and stir for 1 hour. Heat the reaction mass to reflux and stir at reflux for 2 hours. Cool the reaction mass to 30°C and distill out tetrahydrofuran below 40°C. To this reaction mixture add water and ethyl acetate. Separate the organic phase. Wash the organic phase with 10% hydrochloric acid, water, 10% sodium bicarbonate solution and water successively. Dry the organic phase over sodium sulphate and then evaporate the solvent under reduced pressure to get title compound III.

### Step (iii) Preparation of (R)-α-methyl-N-[3-[3-(trifluoromethyl)phenyl]propyl]-1-naphthalene methane amine [cinacalcet base]:

Dissolve 5.0gm of compound-III in tetrahydrofuran and add 17.0gm Lithium aluminium hydride to the solution under nitrogen. Cool the reaction mass to 10°C and stir at 10°C for 2 hours and then at reflux for 2 hrs. Cool the reaction mass to 10°C slowly add water at 10°C. Stir for 30.0 min. Distill out tetrahydrofuran below 40°C. To this reaction mixture add ethyl acetate. Separate the organic phase. Wash the organic phase with 10% sodium bicarbonate solution and water successively. Dry the organic phase over sodium sulphate and then evaporate the solvent under vacuum to get title compound IV.

### Step (iv) Conversion of cinacalcet base into its hydrobromide salt.

Dissolve this cinacalcet free base in acetone and treat with hydrobromic acid solution at room temperature. Stir at room temperature until crystallization occurs. Filter and wash the resulting precipitate with acetone which on air drying yields cinacalcet hydrobromide.

### Example 5 [ Scheme V]

A solution of 3.93g of (R)-(+)-1-(1-Napthyl)ethylamine in 25ml of ethyl acetate is prepared and added gradually over a period of 15 minutes to the reaction mass consisting of 5g of 3-trifluoromethyl cinnamoyl chloride and 9ml of triethylamine in 25ml of ethyl acetate at 10-15°C. After completion of addition, the reaction mass is heated at 45-50°C for 2h. Water is added to this reaction mixture and organic phase separated. The organic phase is washed successively with 10% hydrochloric acid, water, 10% sodium bicarbonate solution and water. The ethyl acetate was distilled to obtain the compound I.

Methanolic solution of 3- (Trifluoromethyl) cinnamic acid (3.0gm in 27ml of methanol) is hydrogenated (5.0kg/cm² of H₂) in the presence of 0.3gm palladium on carbon (10% w/w of compd II) for 3 hours at room temperature. The catalyst is filtered and the solvent is evaporated to dryness to give 2.6g of titled compound-II.[ Cinacalcet base]

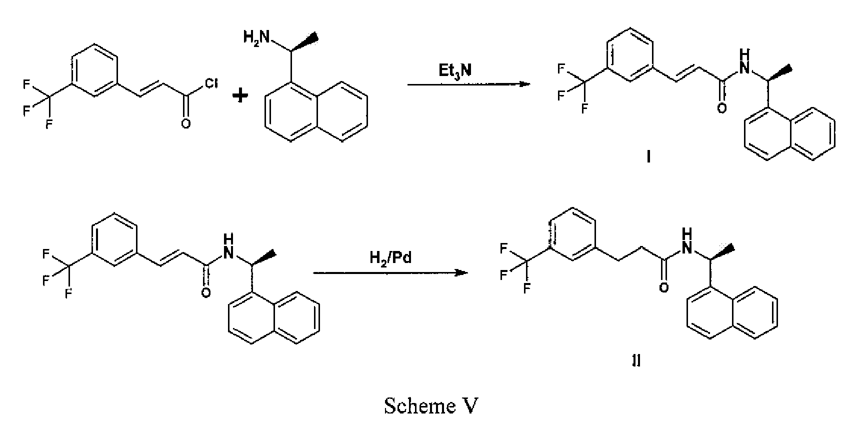

### Example 6 [ Scheme IV]

20.0gm of 3-trifluoromethyl cinnamic acid is dissolved in ethyl acetate and 10.2gm of triethylamine is added. The reaction mass is stirred for 15.0 min. A solution of 9.9 gm of ethyl chloroformate in ethyl acetate is prepared and this solution is slowly added in the reaction mass at 0-5°C. The reaction mass is stirred at 0-5°C for 2 hours to give compound-I as an in situ intermediate in solution form.

A solution of 17.2gm of (R)-(+)-1-(1-Napthyl) ethyl amine in ethyl acetate is prepared and added to the reaction mass at 0-5°C. The reaction mass is stirred at 0-5°C for 1 hour. Water is added to this reaction mixture and organic phase is separated. The organic phase is washed with 10% hydrochloric acid, water, 10% sodium bicarbonate solution and water successively. The organic phase is dried over sodium sulphate and the solvent is evaporated under vacuum to give crude titled compound-II. To this residue, n-hexane was added and heated the reaction mass to reflux for 30.0 min, cooled gradually to room temperature and stirred for 30.0 min. The residue is filtered and washed with chilled n-hexane. Wet compound on drying yields 25.0 gm of titled compound-II.

Methanolic solution of Compound II(20gm in 200ml of methanol) is hydrogenated (5.0kg/cm² of H₂) in the presence of 2gm palladium on carbon (10% w/w of compd III) for 3 hours at room temperature. Then the catalyst was filtered out and the solvent is evaporated to dryness to give 19g of titled compound-III.

### Preparation of (R)-α-methyl-N-[3-[3-(trifluoromethyl)phenyl]propyl]-1- naphthalene methane amine [Cinacalcet base]:

Compound-III (15.0g) is dissolved in tetrahydrofuran and 7.5g of sodium borohydride was added to the solution. The reaction mass is cooled to 5°C and 30.0mL of boron trifluoride-etherate is added drop wise. The reaction mass is then heated to 30°C and stirred for 20 h. The reaction mass is quenched in 10% aqueous hydrochloric acid solution. The reaction mass is then refluxed for 2 h and cooled to 30°C. To this reaction mixture ethyl acetate is added and phase separation is performed. The organic phase is washed with water, 10% sodium bicarbonate solution and water successively. The organic phase is dried over sodium sulphate and the solvent is evaporated to dryness to obtain cinacalcet free base.

### Conversion of cinacalcet base into its hydrochloride salt.[Compound IV]

The cinacalcet free base is dissolved in diisopropyl ether and treated with 1M hydrochloric acid in diethyl ether. The resulting precipitate is collected, washed with diisopropyl ether and air dried to afford 8.8 g of titled compound-IV (cinacalcet hydrochloride).

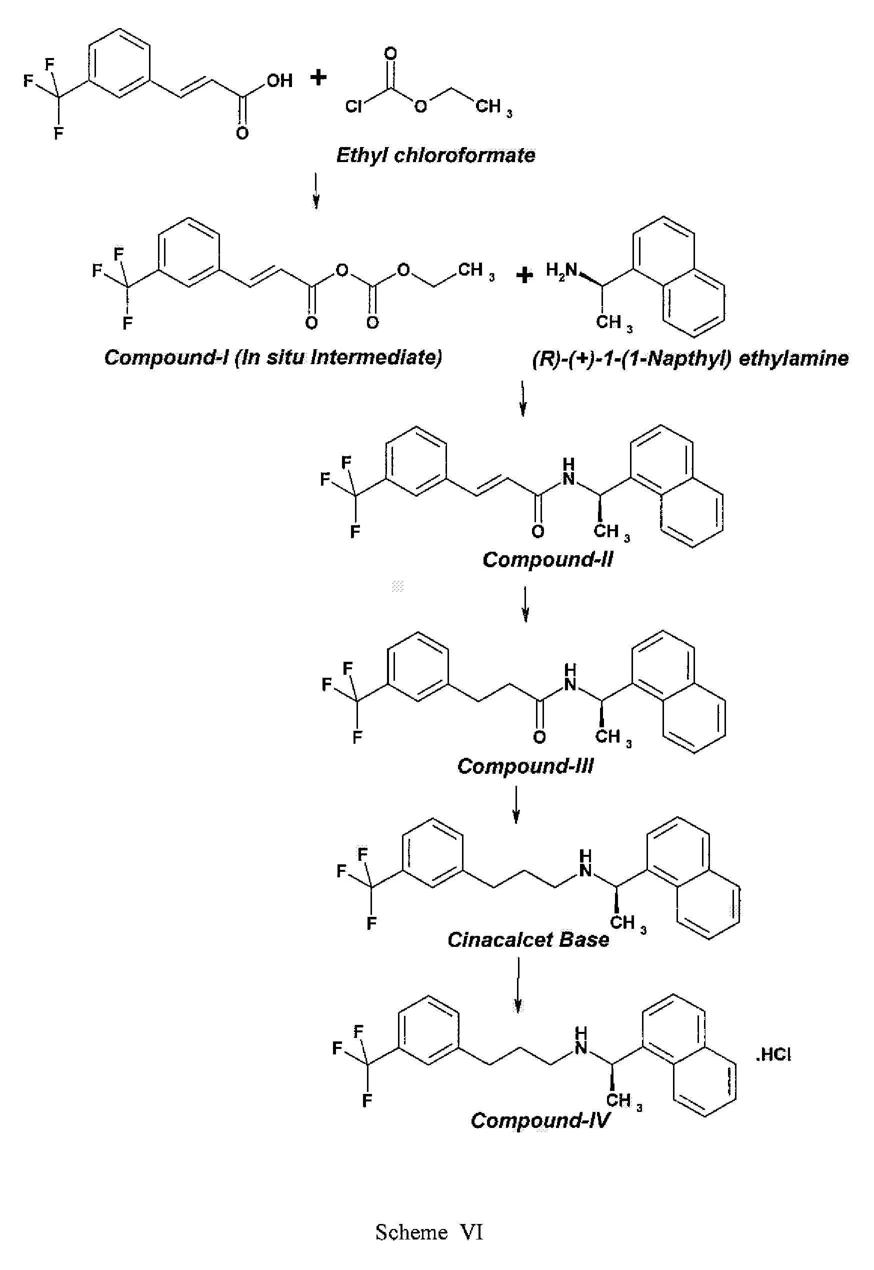

## Claims

1. A process for the preparation of cinacalcet hydrochloride comprising:
(a)hydrogenating 3- (trifluoromethyl) cinnamic acid in the presence of Pd/C, hydrogen and solvent to form 3-trifluoromethylphenyl propionic acid;
(b) reacting -(trifluoromethylphenyl) propionic acid with ethyl chloroformate to give an in-situ intermediate;
(c) condensing the in-situ intermediate with R-(+)-1-(1-naphthyl)ethylamine in a solvent to obtain the corresponding amide;
(d) reducing the amide of step (c) using a reducing agent in a solvent to form the corresponding amine; and
(e) treating the amine of step (d) with HCl in a solvent to form cinacalcet hydrochloride.

2. The process of claim 1 wherein the solvent of step (a) is methanol, ethanol, tetrahydrofuran

3. The process of claim 1 wherein the step (a) is carried out at a pressure of about 1-10 kg/cm² of hydrogen.

4. The process of claim 1 wherein the solvent of step (b) is ethyl acetate, tetrahydrofuran.

5. The process of claim 1 wherein the base of step (b) is triethyl amine, isopropyl ethyl amine, 4-(dimethyl amino) pyridine, potassium carbonate.

6. The process of claim 1 wherein the reducing agent of step (d) is sodium borohydride and boron trifluoride-etherate, sodium borohydride -iodine, lithium aluminum hydride- tetrahydrofuran, sodium borohydride-phosphorous trichloride or sodium borohydride -acetic acid

7. The process of claim 6 wherein the reducing agent is sodium borohydride and boron trifluoride-etherate.

8. The process of claim 1 wherein the solvent of step (d) is tetrahydrofuran.

9. The process of claim 1 wherein the temperature range for step (d) is maintained between 25-80°C.

10. The process of claim 1 wherein the reaction mixture of step (d) is maintained for about 2 hours to 25 hours.

11. The process of claim 1 wherein the solvent of step (e) is diethylether or diisopropylether.

12. The process of claim 1 wherein the amide of step (c) is the following compound

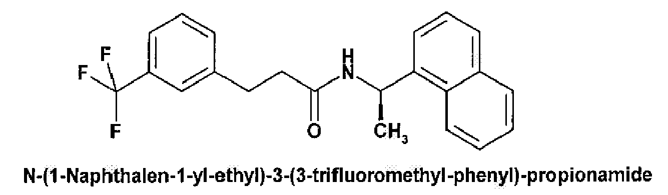

13. The process of claim 1 wherein the *in situ* intermediate of step (b) is the following compound
